(19) Europäisches Patentamt

European Patent Office

Office européen des brevets

(11) Veröffentlichungsnummer: **0 299 289 B1**

(12) **EUROPÄISCHE PATENTSCHRIFT**

(45) Veröffentlichungstag der Patentschrift: **04.03.92**

(21) Anmeldenummer: **88110456.6**

(22) Anmeldetag: **30.06.88**

(51) Int. Cl.5: **C07C  45/40**, //C07D317/54

(54) **Verfahren zur kontinuierlichen Ozonisierung von ungesättigten organischen Verbindungen.**

(30) Priorität: **08.07.87 DE 3722566**

(43) Veröffentlichungstag der Anmeldung:
**18.01.89 Patentblatt  89/03**

(45) Bekanntmachung des Hinweises auf die Patenterteilung:
**04.03.92 Patentblatt  92/10**

(84) Benannte Vertragsstaaten:
**DE**

(56) Entgegenhaltungen:
EP-A- 0 028 295          EP-A- 0 034 738
EP-A- 0 103 144          DE-A- 2 713 863
DE-A- 2 754 366          DE-C- 962 078

CHEMICAL ABSTRACTS, Band 100, Nr. 11, 12. März 1984, Seite 539, Spalte 1, Zusammenfassungsnr. 85617n, Columbus, Ohio, US; L. LI: "Application of ozonization method in perfume production"

CHEMICAL ABSTRACTS, Band 82, Nr. 17, 28. April 1975, Seiten 505-506, Zusammenfassungsnr. 111981h, Columbus, Ohio, US; N.M. BARANOVA et al.: "Effect of the concentration of isosafrole and oxygen on the degree of isosafrole conversion to heliotropin"

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Schulz, Paul, Dr.**
**Auf dem Scheidt 35**
**W-5600 Wuppertal 1(DE)**
Erfinder: **Virnig, Michael Joseph, Dr.**
**1105 Lanewood Way**
**Santa Rosa, 95404 California(US)**
Erfinder: **Carduck, Franz Josef, Dr.**
**Landstrasse 18**
**W-5657 Haan(DE)**

EP 0 299 289 B1

## Beschreibung

Die Erfindung betrifft ein Verfahren zur kontinuierlichen Ozonisierung von ungesättigten organischen Verbindungen, insbesondere Olefinen, bei Temperaturen von höchstens 40°C in einer Reaktionskolonne, in der die ungesättigten Verbindungen in Lösung in einem protischen Lösemittel, in dem die Ozonisierungsprodukte löslich sind, sowie ein Ozon enthaltendes inertes Trägergas im Gleichstrom von oben nach unten geführt werden, wobei zur Abführung der Reaktionswärme ein inertes Kühlmittel in die Reaktionskolonne eingespeist wird.

Verfahren der vorstehend genannten Art, bei denen die Innenkühlung nach Art einer Verdunstungskühlung mit oberhalb der Ozonisierungstemperatur siedenden Kühlmitteln, z. B. Wasser oder wasserhaltigen Emulsionen, vorgenommen wird, sind aus den DE-A 27 13 863, 27 54 366 und 30 05 514 bekannt. Diese Verfahren weisen u. a. den Nachteil auf, daß sie für den Niedrigtemperaturbereich, z. B. Ozonisierungsprozesse, die unterhalb von - 10°C stattfinden (bei Olefinkomponenten, die bei höheren Temperaturen Neben- oder Folgereaktionen eingehen können) wenig geeignet sind. Der Verdunstungskühlprozeß setzt zudem voraus, daß das Ozon/Trägergas-Verhältnis nicht zu hoch ist, um die erforderliche, auf den Ozoneinsatz bezogene Verdunstungs-bzw. Kühlkapazität zu gewährleisten (niedrige Ozonkonzentrationen).

Niedrigtemperaturozonisierungen von ungesättigten Verbindungen können, u. a. im kleinen Maßstab, in gekühlten Mantelkolonnen mit geeigneter Packung durchgeführt werden. Diese Reaktionskolonnen verursachen aber, insbesondere auch durch erforderliche zusätzliche Wärmeaustauscheinheiten, hohe Kosten.

Weiter Nachteile sind:
ein Hauptteil der Reaktionswärme wird auch bei optimaler Auslegung der Kolonne im Hinblick auf die Verweilzeit in einem eng begrenzten Bereich der Kolonne frei. Die "Streckung" der Reaktionszonen durch mehrere Einleitstellen für Olefin bzw. Ozon kann lokale Überschreitungen der Solltemperatur nicht vollständig vermeiden;
die Maßstabsvergrößerung der Reaktionskolonnen führt infolge des zunehmenden Mißverhältnisses von abzuführender Wärmemenge zu verfügbarer Wärmeaustauschkapazität zu erheblichen Auslegungsproblemen;
unvermeidliche punktuelle Überschreitungen der Solltemperatur führen zu Ausbeuteverlusten.

Die vorstehend genannten Probleme werden u. a. im Fall der Verdunstungskühlung noch dadurch verstärkt, daß neuerdings Ozongeneratoren zur Verfügung stehen, die einen Trägergasstrom mit hohen Ozonkonzentrationen, z.B. mit einer Konzentration von 100 g Ozon pro m$^3$ Trägergas, liefern.

Erfindungsgemäß lassen sich die vorstehend genannten Nachteile durch ein Verfahren vermeiden, bei dem die interne Kühlung nach Art einer Verdampfungskühlung durchgeführt wird, d.h. unter Einsatz eines Kühlmittels, dessen Siedetemperatur unterhalb der Ozonisierungstemperatur in der Kolonne liegt.

Das Kühlmittel verläßt die Kolonne (dampfförmig) im Abgasstrom und kann, entsprechend einer bevorzugten Ausführungsform der Erfindung, bei nicht zu niedrigem Siedepunkt durch Kompression/ Kühlung kondensiert und in den Prozeß rückgeführt werden. Extrem niedrig siedende, preiswerte Kühlkomponenten wie Flüssigstickstoff können - in Abhängigkeit von wirtschaftlichen Erwägungen - ebenfalls eingesetzt werden. Eine Rückführung wird in diesem Fall nicht vorgesehen.

Demgemäß ist des Verfahren der Erfindung dadurch gekennzeichnet, daß man als Kühlmittel eine bei Umgebungstemperatur gasförmige oder flüssige Verbindung mit einem Siedepunkt unterhalb + 20°C verwendet, wobei der Siederpunkt des Kühlmittels vorzugsweise mindestens 5°C unterhalb der Ozonisierungstemperatur liegt. Bevorzugt siedet das verwendete Kühlmittel in einem Bereich von 10 bis 30°C unterhalb der Ozonisierungstemperatur; es weist weiterhin vorzugsweise Siedepunkt zwischen - 10°C und - 50°C auf.

Bevorzugte, in dem Verfahren der Erfindung verwendete Kühlmittel sind aus der von Kohlenwasserstoffen, Halogenkohlenwasserstoffen, insbesondere chloriertem und/oder fluoriertem Methan oder Ethan und Dimethylether sowie Kohlendioxid oder Flüssigstickstoff gebildeten Gruppe ausgewählt. Besonders vorteilhaft sind Dichlordifluormethan und - insbesondere bei wertvollen Reaktionsprodukten wie Riechstoffen - flüssiges Kohlendioxid.

Gemäß einer vorteilhaften Ausführungsform der Erfindung verwendet man zur Ozonierung einen ozonbeladenen, inerten Trägergasstrom mit hoher Ozonkonzentration, der durch Desorption des an einer anorganischen Masse, z.B. Silicagel, adsorbierten Ozons mit dem Trägergas erzeugt wird. Ein derartiges Verfahren zur Erzeugung von Ozon ist in der DE-A 32 30 922 beschrieben.

Gemäß einer weiteren vorteilhaften Ausführungsform der Erfindung gibt man das Kühlmittel in die Reaktionskolonne über eine Verteilerdüse am Kolonnenkopf und das Gemisch aus ungesättigten organischen Verbindungen und Lösemittel sowie das Ozon/Trägergas-Gemisch unterhalb des Aufgabeortes des Kühlmittels auf.

Das am Boden der Reaktionskolonne abgezogene Reaktionsgemisch wird in üblicher Weise auf die Ozonisierungsprodukte aufgearbeitet; Kühlmittel und Lösemittel können nach entsprechender Reinigung in den Prozeß zurückgeführt werden, sofern

sich dies aus wirtschaftlichen Gründen empfiehlt.

Zur Durchführung des Verfahrens der Erfindung wird das zu ozonisierende Olefin in einem protischen Lösemittel, insbesondere niederen Alkoholen oder Carbonsäuren, z. B. Methanol, Ethanol, Isopropanol, Isobutanol, Ameisensäure, Essigsäure oder Propionsäure und dergleichen, die sowohl das Ausgangsolefin als auch die Ozonisierungsprodukte bei den Verfahrensbedingungen zu lösen vermögen, auf die Kolonne gegeben; gleichzeitig wird das ozonhaltige Trägergas im Gleichstrom durch die Kolonne geleitet. Oberhalb der Einleitstelle für Olefin und Ozon wird das Verdampfungskühlmittel zugegeben; dabei wird der Durchsatz so eingestellt, daß die Reaktionsenthalpie durch Verdampfung aufgefangen wird und die Temperatur in der Reaktionskolonne sich bei einem vorgegebenen Sollwert stabilisiert. Die am Kolonnenboden abgenommene Phase wird wie angegeben aufgearbeitet. Während der Ozonisierung kann die Kolonne zusätzlich extern leicht gekühlt werden, um Kälteverluste an die Umgebung zu kompensieren.

Das Verfahren der Erfindung bietet den Vorteil, daß es problemlos maßstabvergrößert durchgeführt werden kann, die Abführung der Reaktionswärme unmittelbar aus der Reaktionszone unter Ausschluß von Überhitzungen erfolgt und die erzielten Ausbeuten höher als bei einer indirekten Kolonnenkühlung oder Verdunstungskühlung sind, wobei weniger Nebenprodukte gebildet werden.

Die Erfindung wird im folgenden anhand von Beispielen näher erläutert.

Beispiel 1.

Durch eine 2 m x 40 mm Reaktionskolonne (Edelstahl; MELLAPACK-Füllung) wurden pro Stunde 4.222 g 9%-ige methanolische Isosafrollösung und 115g Ozon (als ca. 1,1 m$^3$/h ca. 8%-iges Ozon/Stickstoff-Gemisch) eingeleitet. Oberhalb der Eintrittsstelle der Olefinlösung wurden durch eine Vollkegeldüse etwa 6,5 kg/h Dichlordifluormethan (Kp. -29,8°C) als Verdampfungskühlmedium auf die Kolonnenpackung verteilt. Die Temperatur wurde über den Kühlmitteldurchsatz gesteuert und bei ca. -10°C konstant gehalten.

Nach katalytisch-reduktiver Aufarbeitung des Ozonisierungsproduktes auf Heliotropin erhielt man eine Ausbeute von ca. 85 -90 %, bezogen auf eingesetztes Isosafrol.

Eine einstufige Kompression und Kühlung des Dichlordifluormethan/Stickstoff/Methanol-Gemisches ergab einen Kühlmittelrückstrom, der neben dem Dichlordifluormethan höchstens ca. 5 % Methanol enthielt und direkt auf die Reaktionskolonne zurückgegeben werden konnte.

Beispiel 2.

Durch die Reaktionskolonne gemäß Beispiel 1 wurden in der Stunde 380 g Isosafrol als 9%-ige methanolische Lösung im Gleichstrom mit 115 g (als 8 m$^3$/h Ozon/Luft-Gemisch) durchgeleitet. Zu Vergleichszwecken wurde dieser Versuch in drei Versionen durchgeführt:

1. Mit Kolonnen-Mantelkühlung (gemäß Stand der Technik).

Der Säulen-Doppelmantel war in vier Kühlzonen unterteilt, die mit -30°C-Kühlsole durchströmt wurden. Der Kältermittel-Durchsatz wurde in jeder Zone so eingestellt, daß die Innentemperatur der Kolonne über die gesamte Länge bei -10°C lag. Dabei wurden ca. 90% der gesamten Kühlsole in der Einleitzone des Olefins verbraucht.

2. Mit Kolonnen-Mantelkühlung und Olefin-Einleitung in vier Punkten der Kolonne (gemäß Stand der Technik).

Die vier Kühlzonen wurden mit gleichem Kühlmitteldurchsatz beschickt. Die Isosafrol-Lösung wurde unmittelbar oberhalb des jeweiligen Kolonnenschusses auf die Säulenpackung über eine Verteilerdüse aufgegeben. Bei voller Kühlleistung wurde die Olefinlösung so auf die vier Einleitstellen verteilt, daß die Innentemperatur der Kolonne an jedem Punkt der Säule maximal -10°C betrug.

3. Mit interner Verdampfungskühlung durch flüssiges Kohlendioxid (erfindungsgemäß).

Die Isosafrol-Lösung wurde i einer Menge von 4,22 kg/h über einer Verteilerdüse eingepumpt und unmittelbar auf der Säulenpackung verteilt. Oberhalb der Olefineinleitstelle befand sich eine weitere Verteilerdüse, durch die ca. 5,6 kg/h Flüssigkohlensäure in den Ozon/Trägergas-Strom eingebracht wurden. Das flüssige Kühlmittel wurde so dosiert, daß die Temperatur der Reaktionszone (ein etwa 25 cm langer Bereich der Kolonne ab Einleitstelle der Reaktanden) sich bei -10°C stabilisierte.

Durch leichte Kühlung mit einer Kühlsole von -12°C im Doppelmantel wurde die Temperatur auch in den restlichen Kolonnenzonen bei -10°C gehalten.

Aus dem Abgas wurde durch Kompression und Kühlung ein Kohlendioxid/Methanol-Gemisch mit einem Gehalt von ca. 1 - 3 % Methanol kondensiert und in die Kühlzone zurückgeführt.

Nach katalytisch-reduktiver Aufarbeitung auf Heliotropin ergaben sich folgende Ausbeuten:
Version 1: ca. 75 %
Version 2: ca. 80 %
Version 3: ca. 85 - 90 %

Bei Version 3 (Kühlung mit Flüssigkohlendioxid gemäß der Erfindung) läßt sich der Olefin/Ozon-Durchsatz unter zuverlässiger Konstanthaltung der Prozeßtemperatur bei -10°C in der beschriebenen Anlage im Gegensatz zu den Versionen 1 und 2 erheblich steigern.

**Patentansprüche**

1.  Verfahren zur kontinuierlichen Ozonisierung von ungesättigten organischen Verbindungen, insbesondere Olefinen, bei Temperaturen von maximal 40°C in einer Reaktionskolonne, in der die ungesättigten Verbindungen in Lösung in einem protischen Lösemittel, in dem die Ozonisierungsprodukte löslich sind, sowie ein Ozon enthaltendes inertes Trägergas im Gleichstrom von oben nach unten geführt werden, wobei zur Abführung der Reaktionswärme ein inertes Kühlmittel in die Reaktionskolonne eingespeist wird, dadurch gekennzeichnet, daß man als Kühlmittel eine bei Umgebungstemperatur gasförmige oder flüssige Verbindung mit einem Siedepunkt im Bereich von +20°C bis -200°C verwendet, wobei der Siedepunkt des Kühlmittels mindestens 5°C unterhalb der Ozonisierungstemperatur liegt.

2.  Verfahren nach Anspruch 1, dadurch gekennzeichnet, daß als Kühlmittel Flüssig-Stickstoff eingesetzt wird.

3.  Verfahren nach Anspruch 1, dadurch gekennzeichnet, daß man als Kühlmittel eine Verbindung mit einem Siedepunkt im Bereich von +20°C bis -100°C verwendet.

4.  Verfahren nach Anspruch 1 oder 3, dadurch gekennzeichnet, daß als Kühlmittel einen Siedepunkt im Bereich von 10°C bis 30°C unterhalb der Ozonisierungstemperatur aufweist.

5.  Verfahren nach Anspruch 1, 3 oder 4, dadurch gekennzeichnet, daß der Siedepunkt des verwendeten Kühlmittels zwischen -10°C und -50°C liegt.

6.  Verfahren nach einem der Ansprüche 1 sowie 3 bis 5, dadurch gekennzeichnet, daß man ein Kühlmittel verwendet, das aus der von Kohlenwasserstoffen, Halogenkohlenwasserstoffen, insbesondere chloriertem und/oder fluoriertem Methan oder Ethan und Dimethylether sowie Kohlendioxid gebildeten Gruppe ausgewählt ist.

7.  Verfahren nach einem der Ansprüche 1 bis 6, dadurch gekennzeichnet, daß man die Ozonierung mit einem ozonbeladenen inerten Trägergasstrom durchführt, der durch Desorption des an einer anorganischen Masse adsorbierten Ozons mit dem Trägergas erzeugt wird.

8.  Verfahren nach einem der Ansprüche 1 bis 7, dadurch gekennzeichnet, daß man das Kühlmittel in die Reaktionskolonne über eine Verteilerdüse am Kolonnenkopf und das Gemisch aus ungesättigten organischen Verbindungen und Lösemittel sowie das Ozon/Trägergas-Gemisch unterhalb des Aufgabeortes des Kühlmittels aufgibt.

**Claims**

1.  A process for the continuous ozonization of unsaturated organic compounds, particularly olefins, at temperatures of at most 40°C in a reaction column through which the unsaturated compounds - in solution in a protic solvent in which the ozonization products are soluble - and an ozone-containing inert carrier gas are passed downwards in co-current, an inert coolant being fed into the reaction column to dissipate the heat of reaction, characterized in that the coolant used is a compound gaseous or liquid at ambient temperature with a boiling point in the range from +20°C to -200°C, the boiling point of the coolant being at least 5°C below the ozonization temperature.

2.  A process as claimed in claim 1, characterized in that liquid nitrogen is used as the coolant.

3.  A process as claimed in claim 1, characterized in that a compound having a boiling point in the range from +20°C to -100°C is used as the coolant.

4.  A process as claimed in claim 1 or 3, characterized in that the coolant has a boiling point in the range from 10°C to 30°C below the ozonization temperature.

5.  A process as claimed in claim 1, 3 or 4, characterized in that the boiling point of the coolant used is between -10°C and -50°C.

6.  A process as claimed in any of claims 1 and 3 to 5, characterized in that the coolant used is selected from the group comprising hydrocarbons, halogenated hydrocarbons, more especially chlorinated and/or fluorinated methane or ethane, and dimethyl ether and also carbon dioxide.

7.  A process as claimed in any of calims 1 to 6, characterized in that the ozonization is carried out with an inert carrier gas stream charged with ozone which is produced by desorption of the ozone adsorbed on an inorganic material with the carrier gas.

**8.** A process as claimed in any of claims 1 to 7, characterized in that the coolant is introduced into the reaction column through a distributor nozzle at the head of the column while the mixture of unsaturated organic compounds and solvent and also the ozone/carrier gas mixture are introduced beneath the coolant inlet.

**Revendications**

**1.** Procédé d'ozonation en continu de composés organiques insaturés, notamment d'oléfines à des températures de 40° au plus dans une colonne de réaction dans laquelle on introduit de haut en bas les composés insaturés qui sont en solution dans un solvant protique, dans lequel les produits d'ozonation sont solubles, ainsi qu'un gaz porteur inerte contenant de l'ozone dans le même sens, en injectant dans la colonne de réaction pour éliminer la chaleur de réaction un réfrigérant inerte, caractérisé en ce qu on utilise comme réfrigérant un composé liquide ou gazeux à la température environnante avec un point d'ébullition compris entre + 20°C et -200°C, le point d'ébullition du réfrigérant se trouvant au moins à 5°C en-dessous de la température d'ozonation.

**2.** Procédé selon la revendication 1, caractérisé en ce qu'on utilise comme réfrigérant de l'azote liquide.

**3.** Procédé selon la revendication 1, caractérisé en ce qu'on utilise comme réfrigérant un composé avec un point d'ébullition compris entre +20°C et -100°C.

**4.** Procédé selon la revendication 1 ou 3, caractérisé en ce que le produit utilisé comme réfrigérant présente un point d'ébullition dans le domaine de 10° à 30°C en-dessous de la température d'ozonation.

**5.** Procédé selon la revendication 1, 3 ou 4, caractérisé en ce que le point d'ébullition du réfrigérant utilisé se trouve entre -10°C et -50°C.

**6.** Procédé selon l'une des revendications 1, ainsi que 3 à 5, caractérisé en ce qu'on utilise un réfrigérant qu'on choisit dans le groupe formé par les hydrocarbures, les hydrocarbures halogénés notamment le méthane ou l'éthane chloré et/ou fluoré et l'éther diméthylique, ainsi que le dioxyde de carbone.

**7.** Procédé selon l'une des revendications 1 à 6, caractérisé en ce qu'on réalise l'ozonation avec un courant de gaz porteur inerte chargé d'ozone, qu'on prépare par désorption dans le gaz porteur de l'ozone adsorbé sur une masse minérale.

**8.** Procédé selon l'une des revendications 1 à 7, caractérisé en ce qu'on introduit le réfrigérant dans la colonne de réaction par une buse de répartition en tête de colonne et le mélange de composés organiques insaturés et de solvant, ainsi que le mélange ozone/gaz porteur en-dessous de l'endroit d'introduction du réfrigérant.